# EUROPÄISCHE PATENTANMELDUNG

(11) **EP 3 090 687 A1**
(43) Veröffentlichungstag der Anmeldung: **09.11.2016**
(21) Anmeldenummer: 15166306.9
(22) Anmeldetag: 04.05.2015
(51) Int. Cl.: A61B 17/00, A61B 17/34, A61B 90/00

(54) **EINFÜHRUNGS- UND POSITIONIERVORRICHTUNG FÜR PUNKTIONSNADELN, ZUGEHÖRIGER BAUSATZ UND ZUGEHÖRIGE VERFAHREN**

(71) Anmelder: Medical Templates AG, 8132 Egg bei Zürich (CH)
(72) Erfinder: Die Erfindernennung liegt noch nicht vor
(74) Vertreter: Schmauder & Partner AG Patent- & Markenanwälte VSP

(57) **Zusammenfassung**

Eine Einführungs- und Positioniervorrichtung für mindestens eine Punktionsnadel weist ein oberes Plattenelement (2) und ein unteres Plattenelement (4) auf, welche mittels Verbindungselementen (6) starr voneinander beabstandet gehalten sind. Jedes Plattenelement ist mit einer Vielzahl von Durchgangslöchern (8) zur Durchführung einer Punktionsnadel vorgegebener Aussenabmessungen versehen, wobei ein jedes Durchgangsloch mit Anpassungsmitteln ausgestattet sind, um eine Querabmessung des Durchgangsloches an einen durch den gewählten Neigungswinkel definierten Aussenquerschnitt der Punktionsnadel anzupassen. Die Vorrichtung weist zudem in einem tomografischen Bildgebungsverfahren erkennbare Markierungsmittel zur Bestimmung der räumlichen Lage und Ausrichtung der Einführungs- und Positioniervorrichtung auf.

## Beschreibung

### Technisches Gebiet

Die Erfindung betrifft eine Einführungs- und Positioniervorrichtung für mindestens eine Punktionsnadel gemäss Oberbegriff des Anspruchs 1. Ausserdem betrifft die Erfindung: einen Bausatz zur Einführung und Positionierung mindestens einer Punktionsnadel; ein Verfahren zur Planung der Einführung und Positionierung mindestens einer Punktionsnadel in eine vordefinierte Eingriffsregion eines Patienten; sowie ein Verfahren zur Einführung und Positionierung mindestens einer Punktionsnadel in eine vordefinierte Eingriffsregion eines Patienten.

### Stand der Technik

Es gibt mehrere medizinische Situationen, in denen ein kontrolliertes Einführen und Positionieren einer Punktionsnadel an einer bestimmten Stelle im Körper eines Patienten erforderlich ist. Als Punktionsnadeln kommen insbesondere Biopsienadeln zur Entnahme von Gewebeproben zum Einsatz. Daneben kommen auch Injektionsnadeln zur kontrollierten Verabreichung einer Substanz in einer bestimmten Region im Körperinneren sowie Führungsnadeln für Operationsschrauben oder -bohrer in Frage. Bei all diesen Anwendungen gilt es, einen definierten Teil der Punktionsnadel - primär deren Spitze - an einer gewünschten Stelle im Körperinneren zu positionieren. Darüber hinaus ist eine kontrollierte Einführung der Punktionsnadel erforderlich, d.h. auch die Einstichstelle an der Körperoberfläche, sowie der Stichkanal werden aufgrund verschiedener Kriterien gewählt.

Wenn eine möglichst präzise Einführung und/oder Positionierung der Punktionsnadel erforderlich ist, wird der Vorgang unter Zuhilfenahme von bildgebenden Verfahren durchgeführt. Zu diesem Zweck sind bereits mehrere Einführungs- und Positioniervorrichtungen vorgeschlagen worden. Als bildgebende Verfahren werden vornehmlich Magnetresonanztomografie (MRT), Röntgentomographie (CT), 3D Röntgen, sowie die Fluoroskopie eingesetzt.

Die EP 0 640 842 A1 (Philips) beschreibt eine MRT gestützte Biopsievorrichtung, die insbesondere für mammografische Untersuchungen vorgesehen ist. Die Vorrichtung umfasst eine mit einem Raster von Durchgangslöchern versehene Platte, welche eine x-y-Ebene definiert und durch welche eine Biopsienadel in z-Richtung vorgetrieben werden kann. Durch Wahl eines bestimmten Durchgangslochs für die Nadel ist eine grobe x-y-Positionierung möglich. Falls die x-y-Position genauer als der Lochabstand eingestellt werden soll, kann die ganze Platte mittels einer Stellvorrichtung kontinuierlich in der x-y-Ebene verschoben werden. Die Vorrichtung umfasst weiterhin mindestens ein stabförmiges Phantom, das in einer bekannten Ausrichtung gegenüber dem Gehäuse fixiert ist. Das Phantom besteht aus einem im MRT sichtbaren Material und dient als Positionierhilfe für die Biopsievorrichtung. Vorteilhafterweise ist das Phantom als orthogonales Kreuz ausgebildet, dessen Arme beispielsweise in x- bzw. y-Richtung verlaufen. In einer Ausführungsform ist zudem die Biopsienadel im Bereich der Spitze mit einem weiteren Phantom ausgestattet, mit dessen Hilfe die z-Position der Nadelspitze im MRT-Bild bestimmbar ist. Auch die in der WO 99/058069 (Tomo-Vision) beschriebene Punktionsvorrichtung für Schichtaufnahmeverfahren beruht auf der Verwendung von länglichen Hohlräumen, die mit einem entsprechend gewählten Kontrastmittel gefüllt sind. Dadurch, dass eine Mehrzahl solcher Hohlräume mit unterschiedlicher Richtung und Versatz vorhanden ist, lässt sich anhand der in einer Schichtaufnahme erkennbaren Schnitte der Hohlräume die Position und Neigung der Punktionsvorrichtung bestimmen. Die EP 2409645 A1 sowie die DE 10 2011 080 682 A1 (Siemens) betreffen weitere bildgebungsunterstützte Biopsievorrichtungen, welche eine steuerbare Einheit für die Positionierung und den Vortrieb einer Biopsienadel umfassen. Auch in diesen Fällen kommt eine Positioniereinheit zum Einsatz, die einen kontrollierten Nadelvortrieb in z-Richtung und eine Wahl der Nadelposition in einer dazu senkrechten x-y-Ebene erlaubt. Ein solcher Ansatz ist gerade bei mammografischen Biopsievorrichtungen zweckmässig, weil die zu untersuchende Körperregion zwischen zwei Platten angeordnet und somit pseudoflächig geformt ist. Dementsprechend ist der Zugang zu einer bestimmten Stelle vorteilhaft durch einen Einstich quer zur Plattenebene möglich. Es gibt jedoch zahlreiche andere Situationen, in denen ein direkter Einstich auf dem kürzesten Weg senkrecht zur Hautoberfläche wegen dazwischen liegender Strukturen nicht möglich oder zumindest suboptimal ist.

Einen Ansatz mit wählbarem Einstichwinkel ist in der US 6249713 B1 (Siemens) beschrieben. Um die Spitze einer Biopsienadel von einer vorgegebenen Einstichstelle an eine gewünschte Stelle im Körperinnern zu bringen, kann die Richtung der Nadelachse eingestellt werden. Insbesondere kann diese Richtung in an sich bekannter Weise durch einen Polarwinkel und einen Azimutwinkel definiert werden. Allerdings ist die Prozedur wie auch die betreffende Vorrichtung vergleichsweise aufwändig.

Eine weitere Art von Biopsienadelführung ist in der US 2008/0146963 (Crocker) beschrieben. Diese umfasst zwei endständig miteinander verbundene bandförmige Elemente, die je mit einer Vielzahl von Durchgangslöchern für eine Biopsienadel versehen sind. Das erste bandförmige Element ist zum anliegenden Befestigen an einem Körperteil eines Patienten vorgesehen, wobei die einzelnen Durchgangslöcher entsprechende Einstichpositionen definieren. Das zweite bandförmige Element ist zwischen den endständigen Verbindungen länger als das erste Element und verläuft dementsprechend nach aufwärts gebogen. Bei gegebener Einstichposition lässt sich die Einstichrichtung durch Wahl eines Durchgangsloches im oberen Bandelement approximativ festlegen. Wegen der Biegsamkeit des oberen Bandelementes liegt jedoch keine eigentliche Führung vor, sondern vielmehr eine Ausrichtungshilfe.

### Darstellung der Erfindung

Aufgabe der Erfindung ist es, eine verbesserte Einführungs- und Positioniervorrichtung für mindestens eine Punktionsnadel bereit zu stellen, welche insbesondere frei von den oben erwähnten Nachteilen ist. Eine weitere Aufgabe besteht darin, einen Bausatz zur Einführung und Positionierung mindestens einer Punktionsnadel bereit zu stellen. Noch weitere Aufgaben bestehen darin, ein Verfahren zur Planung der Einführung und Positionierung mindestens einer Punktionsnadel in eine vordefinierte Eingriffsregion eines Patienten sowie ein Verfahren zur Einführung und Positionierung mindestens einer Punktionsnadel in eine vordefinierte Eingriffsregion eines Patienten anzugeben.

Diese Aufgaben werden erfindungsgemäss gelöst durch die Einführungs- und Positioniervorrichtung nach Anspruch 1, durch den Bausatz nach Anspruch 11 sowie durch die Verfahren nach Anspruch 13 bzw. 14.

Vorteilhafte Ausgestaltungen und Ausführungsformen sind in den abhängigen Ansprüchen definiert.

Die erfindungsgemässe Einführungs- und Positioniervorrichtung für mindestens eine Punktionsnadel umfasst ein oberes Plattenelement und ein unteres Plattenelement, welche mittels Verbindungselementen starr voneinander beabstandet gehalten sind, wobei jedes Plattenelement mit einer Vielzahl von Durchgangslöchern zur Durchführung einer Punktionsnadel vorgegebener Aussenabmessungen versehen ist. Durch Wahl eines Durchgangsloches im unteren Plattenelement ist eine Einstichstelle und durch Wahl je eines Durchgangsloches im oberen und im unteren Plattenelement ist zudem ein Neigungswinkel der Punktionsnadel wählbar. Jedes Durchgangsloch ist mit Anpassungsmitteln versehen, um eine Querabmessung des Durchgangsloches an einen durch den gewählten Neigungswinkel definierten Aussenquerschnitt der Punktionsnadel anzupassen. Ausserdem sind in einem tomografischen Bildgebungsverfahren erkennbare Markierungsmittel zur Bestimmung der räumlichen Lage und Ausrichtung der Einführungs- und Positioniervorrichtung vorhanden.

Grundsätzlich ist die erfindungsgemässe Vorrichtung insbesondere für Biopsie- und Injektionsnadeln vorgesehen. Allerdings kann sie auch zur Einführung und Positionierung anderer Arten von Punktionsnadeln im weitesten Sinn wie beispielsweise Führungsnadeln für Schrauben oder Bohrer oder Verstärkungshüllen für Biopsie- und Injektionsnadeln oder sogar für einen Bohrer verwendet werden. Vorteilhafterweise, aber nicht zwangsläufig, haben die verwendeten Nadeln zumindest im Bereich, der von der erfindungsgemässen Vorrichtung geführt werden soll, ein im Wesentlichen konstantes Aussenprofil. Es versteht sich jedoch, dass das Aussenprofil im Bereich der Nadelspitze verjüngt ist. Für zahlreiche Anwendungen haben die Punktionsnadeln ein kreisrundes Aussenprofil. Weiterhin versteht sich, dass die Abmessungen der Durchgangslöcher auf die Aussenabmessungen der jeweiligen Punktionsnadel abgestimmt sein müssen bzw. dass für gegebene Durchgangslöcher nur Punktionsnadeln passender Grössen einsetzbar sind.

Die erfindungsgemässe Vorrichtung ist für den Einsatz in einem tomografischen Bildgebungsverfahren bestimmt, wobei es sich namentlich um MRT, CT, 3D Röntgen sowie CT-Fluoroskopie handelt. Es versteht sich, dass sämtliche Bestandteile der Vorrichtung aus einem Material gebildet sein müssen, das mit dem vorgesehenen Bildgebungsverfahren, gegebenenfalls auch mit mehreren Bildgebungsverfahren, kompatibel ist. Derartige Materialien sind auf dem Fachgebiet bekannt. Insbesondere können geeignete Polymermaterialien wie beispielsweise Polyamid verwendet werden. Ferner muss das Verfahren mit einer ausreichenden räumlichen Auflösung, die insbesondere vergleichbar mit den Abmessungen der Punktionsnadel sein muss, eine dreidimensionale Darstellung der jeweiligen Eingriffsregion liefern können. Hingegen ist es grundsätzlich unerheblich, ob die dreidimensionale Darstellung aus einer Reihe axial versetzter Schnittbilder wie beispielsweise bei der herkömmlichen Röntgentomografie oder bei 2D-MRT-Methoden gewonnen wird, oder mit einem eigentlichen 3D-Verfahren wie der Spiral-CT Technik, der C-Bogen basierten 3D-Röntgenbildgebung oder gewissen 3D-MRT-Methoden.

Der Begriff "Plattenelement" ist im vorliegenden Zusammenhang nicht auf streng planare Gebilde beschränkt. Vielmehr ist darunter ein starres flächiges Objekt zu verstehen, dessen Abmessungen in sämtlichen Flächenrichtungen wesentlich grösser als die Dicke sind. Es kann vorteilhaft sein, das untere Plattenelement leicht konkav auszubilden, um es auf eine konvexe Körperregion eines Patienten anliegend aufsetzen zu können. Das zugehörige obere Plattenelement kann ebenso gekrümmt oder aber planar ausgebildet sein.

Die Vorrichtung weist zudem Markierungsmittel auf, die im vorgesehenen Bildgebungsverfahren erkennbar sind und die zur Bestimmung der räumlichen Lage und Ausrichtung dienen. Demnach dürfen die Markierungsmittel nicht allesamt in einer Ebene angeordnet sein. Es versteht sich, dass die Position der Markierungsmittel an der Vorrichtung bekannt sein muss und vorteilhafterweise in einem zugehörigen Steuerungssystem hinterlegt ist. Im Falle von CT können als Markierungsmittel beispielsweise kleine metallische Einsätze oder aufgedampfte Markierungen verwendet werden. Weiterhin können aber im Falle von CT auch zusätzliche Durchgangslöcher in den Plattenelementen als Markierungsmittel vorgesehen werden. Für MRT geeignete Markierungssubstanzen sind beispielsweise Kupfersulfat, Eisenoxid-Nanopartikel, Vitamin E und Glycerin. Falls die Vorrichtung in einem Material ausgeführt ist, welches vom bildgebenden Verfahren sichtbar erfasst wird, können die Markierungsmittel auch als Aussparungen und/oder Vorsprünge ausgeführt sein.

Die Abmessungen der Vorrichtung richten sich primär nach dem gewünschten Anwendungsbereich und den verwendeten Materialien. Vorzugsweise haben die Plattenelemente eine Kantenlänge von 40 bis 150 mm und sind mit 20 bis 300 Durchgangsöffnungen versehen. Die Dicke der Plattenelemente beträgt vorzugsweise 0.5 mm bis 10 mm. Aus Stabilitätsgründen ist es zweckmässig, wenn die Plattenelemente eine vergleichsweise dicke Randregion, beispielsweise mit einer Dicke von 3 bis 10 mm, und einer vergleichsweise dünnen Innenregion, beispielsweise mit einer Dicke von 0.5 bis 2 mm, zu dimensionieren. Der Abstand zwischen den Plattenelementen beträgt vorzugsweise bis zu 100 mm.

Wenngleich die erfindungsgemässe Vorrichtung zur Einführung und Positionierung einer einzelnen Punktionsnadel verwendbar ist, können damit bei Bedarf auch mehrere Punktionsnadeln eingeführt und positioniert werden. In einem solchen Fall muss für jede Punktionsnadel je ein bestimmtes Durchgangsloch im unteren Plattenelement und ein bestimmtes Durchgangsloch im oberen Plattenelement, nachfolgend auch kurz als "Lochpaar" bezeichnet, verwendet werden.

Dementsprechend umfasst der erfindungsgemässe Bausatz eine erfindungsgemässe Einführungs- und Positioniervorrichtung sowie mindestens eine Punktionsnadel, wobei die Punktionsnadel zumindest abschnittsweise aus einem im tomografischen Bildgebungsverfahren erkennbaren Material gebildet ist. Dadurch lässt sich die momentane Position und Ausrichtung der Punktionsnadel mittels des besagten Bildgebungsverfahrens bestimmen. Unter dem Begriff "zumindest abschnittsweise" fallen auch Ausgestaltungen, bei denen die Punktionsnadel mit angebrachten Marker-Elementen aus einem geeigneten Fremdmaterial versehen ist. Solche Marker-Elemente können fest an der Punktionsnadel fixiert sein; alternativ können sie mit geeigneten Befestigungsmitteln in variablen Positionen längs der Punktionsnadel angebracht, beispielsweise angeklemmt werden. Weiterhin versteht sich, dass die Punktionsnadel aus mit dem tomografischen Bildgebungsverfahren kompatiblen Materialien besteht.

Der Neigungswinkel einer Punktionsnadel bestimmt deren effektiven Aussenquerschnitt in der Ebene des Plattenelementes. Dementsprechend sind für eine gute Führung der Punktionsnadel, aber auch für deren Einführbarkeit bei grossem Neigungswinkel, entsprechende Anpassungsmittel erforderlich, um die wirksame Querabmessung des Durchgangsloches an den jeweiligen Aussenquerschnitt anzupassen.

Gemäss einer vorteilhaften Ausführung (Anspruch 2) ist ein jedes Anpassungsmittel durch mindestens ein elastisches Element im betreffenden Durchgangsloch ausgebildet. Hierbei kann es sich um eine Einlage in Form eines ringartigen Elementes aus einem gummielastischen Material handeln, dessen Innendurchmesser vergleichbar mit der Nennweite der vorgesehenen Punktionsnadeln ist. Damit wird bereits bei senkrechter Nadeldurchführung ein satter Sitz gewährleistet und bei schräger Nadeldurchführung eine elastische Anpassung an den effektiven Nadelquerschnitt gewährt. Alternativ kann das elastische Element durch eines oder mehrere über die Öffnung gespannte elastische Bänder gebildet sein, welche einen Teil der Öffnung freigeben und dabei eine laterale Spannkraft auf eine eingeführte Punktionsnadel ausüben.

Gemäss einer anderen vorteilhaften Ausführung (Anspruch 3) ist ein jedes Anpassungsmittel durch eine sternartige Ausgestaltung des betreffenden Durchgangsloches gebildet. Zweckmässigerweise weist die sternartige Öffnung vier bis zehn Arme, insbesondere 6 Arme auf. Unter dem Begriff "sternartig" sind nebst der Sternform im engeren Sinn auch abgewandelte Formen zu verstehen, bei denen beispielsweise die inneren Vertices durch Kreissegmente ersetzt sind. Die sternartige Ausgestaltung erlaubt einerseits, eine durch die Öffnung geführte Punktionsnadel in einer gewählten Ecke anzulegen und dadurch in einer definierten Position festzuhalten. Besonders vorteilhaft ist dabei, dass die Schenkel der Sternform die Aufnahme von Punktionsnadeln mit unterschiedlichen Aussenabmessungen erlauben. Ein weiterer Vorteil ergibt sich dadurch, dass jeder Sternarm als eigenständiges Durchgangsloch wirken kann. Auf diese Weise wird die Orts- und Winkelauflösung der Einführungs- und Positioniervorrichtung verfeinert.

Vorteilhafterweise (Anspruch 4) sind die einzelnen Durchgangslöcher mit visuellen Identifikationsmitteln versehen, die das Einsetzen der Punktionsnadel in ein zuvor ermitteltes Lochpaar erleichtern. Beispielsweise kann es sich um eine Bezeichnung für Zeilen und Spalten der Lochanordnung handeln, also etwa "B 3" für die zweite Löcherzeile und dritte Löcherspalte. Es können auch weitere Arten von Identifkationsmitteln wie beispielsweise Farbmarkierungen vorgesehen sein. Zur Erleichterung der Erkennung solcher Identifikationsmittel, aber auch generell für eine bessere visuelle Zugänglichkeit des Einstichbereichs ist es zudem von Vorteil, wenn zumindest das obere Plattenelement transparent ist (Anspruch 5).

Grundsätzlich können die Durchgangslöcher eines Plattenelementes verschiedenste Anordnungen haben. Auch ist es grundsätzlich möglich, in den beiden Plattenelementen unterschiedliche Anordnungen zu haben. In aller Regel wird es jedoch bevorzugt sein, beide Plattenelemente mit derselben Lochanordnung auszustatten. Gemäss einer Ausgestaltung (Anspruch 6) sind die Durchgangslöcher als äquidistantes, vorzugsweise orthonormales zweidimensionales Gitter angeordnet.

Grundsätzlich könnte die Vorrichtung schachteiförmig ausgestaltet sein. Alternativ ist eine Anordnung gänzlich ohne Seitenwände möglich, bei der die Verbindungselemente zwischen den beiden Plattenelementen säulenförmig sind. Vorteilhaft ist eine Ausgestaltung, bei der die Verbindungselemente als Seitenwandelemente ausgebildet sind, wobei diese optional mit Ausnehmungen versehen sind (Anspruch 7). Besonders vorteilhaft ist eine Ausgestaltung, bei der die Verbindungselemente jeweils zumindest von einem der Plattenelemente lösbar sind derart, dass die Plattenelemente aufeinander stapelbar sind. Dadurch lässt sich beispielsweise nach anfänglich erfolgter Einführung der Nadel der Plattenabstand verringern und dadurch ein weiteres Vortreiben der Nadel ermöglichen. Zu diesem Zweck können die Verbindungselemente in den Eckbereichen der Plattenelemente angeordnet sein, wobei z.B. jedes Verbindungselement am unteren Plattenelement nach aussen schwenkbar angelenkt und am oberen Plattenelement mit einer seitlich einführbaren Steckverbindung lösbar verbunden ist. Für eine gute Lösbarkeit bei trotzdem guter Stabilität im zusammengesetzten Zustand werden vorteilhafterweise einrastende Verbindungen verwendet.

In gewissen Situationen kann es schwierig oder sogar unmöglich sein, die gesamte Einführungs- und Positioniervorrichtung im Aufnahmebereich eines bildgebenden Tomografiegeräts unterzubringen, sondern nur einen Teil derselben. Deshalb ist es zweckmässig, wenn die erfindungsgemäss vorgesehene Markierungsmittel derart an der Vorrichtung angebracht sind, dass genügend Markierungsmittel in den Aufnahmebereich des Geräts bringbar sind, um daraus die räumliche Lage und Ausrichtung der Vorrichtung bestimmen zu können. Diesbezüglich ist es von Vorteil, wenn die Markierungsmittel als Vorsprünge und/oder Ausnehmungen in einer jeweiligen Seitenfläche eines Plattenelementes und/oder als zusätzliche Durchgangslöcher im Plattenelement ausgestaltet sind (Anspruch 9). Besonders vorteilhaft ist es in einem solchen Fall, wenn das von den Vorsprüngen und/oder Ausnehmungen gebildete Muster für die betreffende Seitenfläche charakteristisch ist und somit eine Identifikation des Vorrichtungsteils im aufgenommenen Bild erlauben. Zu demselben Zweck können die als Markierungsmittel vorgesehenen Durchgangslöcher mit charakteristischen Lochformen und ggf. -grössen realisiert werden.

Vorteilhafterweise sind zudem Fixierungsmittel vorhanden, um die Vorrichtung an einem Patienten zu fixieren (Anspruch 10). Bewährt hat sich insbesondere eine Haftmittelschicht an der Unterseite des unteren Plattenelementes, die mit einer abziehbaren Schutzfolie versehen ist. Damit lässt sich die Vorrichtung an der Körperoberfläche des Patienten lösbar befestigen. Zur Verbesserung der Haftung erstreckt sich die Haftschicht zusätzlich auf laschenartige ausklappbare Elemente, die am Plattenelement angebracht sind. Weitere Fixierungsmittel sind beispielsweise Vakuumverankerungen, aushärtende Materialien oder Gele. Es gibt aber auch Anwendungen, bei denen die Vorrichtung nicht in direkten Kontakt mit dem Patienten gebracht werden soll, sondern durch andere Mittel in einer zwar beabstandeten, aber dennoch wohldefinierten relativen Position festgehalten wird. Beispielsweise für Eingriffe in der Schulterregion wäre es schwierig, die Vorrichtung anliegend am Patienten anzubringen.

Das erfindungsgemässe Verfahren zur Planung der Einführung und Positionierung mindestens einer Punktionsnadel in eine vordefinierte Eingriffsregion eines Patienten, die sich in einem Aufnahmebereich eines bildgebenden Tomografiegeräts befindet, umfasst die folgenden Schritte:
a) Anordnen einer erfindungsgemässen Einführungs- und Positioniervorrichtung derart, dass sich die Vorrichtung in einer fixen Position und Ausrichtung gegenüber der Eingriffsregion befindet;
b) Erfassen einer dreidimensionalen Bilddarstellung mittels des Tomografiegeräts, welche zumindest die Eingriffsregion und die Markierungsmittel der Einführungs- und Positioniervorrichtung enthält;
c) rechnergestützte Bestimmung der relativen räumlichen Lage der Eingriffsregion sowie sämtlicher Durchgangslöcher;
d) rechnergestützte Bestimmung und Ausgabe von zielführenden Kombinationen jeweils eines ersten Durchgangsloches im oberen Plattenelement und eines zweiten Durchgangsloches im unteren Plattenelement, für welche eine die beiden Durchgangslöcher mittig durchlaufende Gerade auf die Eingriffsregion trifft; und
e) optional, rechnergestützte Bestimmung des jeweiligen Abstandes zwischen zweitem Durchgangsloch und Eingriffsregion.

Das erfindungsgemässe Verfahren zur Einführung und Positionierung mindestens einer Punktionsnadel in eine vordefinierte Eingriffsregion eines Patienten, die sich in einem Aufnahmebereich eines bildgebenden Tomografiegeräts befindet, umfasst die folgenden Schritte:
a) Durchführung des erfindungsgemässen Planungsverfahrens;
b) Auswahl einer zielführenden Kombination eines ersten Durchgangsloches im oberen Plattenelement und eines zweiten Durchgangsloches im unteren Plattenelement;
c) Einführung einer Punktionsnadel durch das erste und zweite Durchgangsloch, Einstich in eine an das zweite Durchgangsloch angrenzende Hautregion und Vortrieb der Punktionsnadel bis deren Spitze zur Eingriffsregion gelangt.

Gemäss einer vorteilhaften Ausführungsform (Anspruch 15) ist die Punktionsnadel zumindest abschnittsweise aus einem im tomografischen Bildgebungsverfahren erkennbaren Material gebildet, und der Schritt c) wird mittels eines tomografischen Bildgebungsverfahrens gesteuert. Dadurch lässt sich eine besonders präzise, interaktive Vorgehensweise implementieren. Wie bereits oben erwähnt, umfasst der Begriff "zumindest abschnittsweise" auch Ausgestaltungen, bei denen die Punktionsnadel mit angebrachten Marker-Elementen aus einem geeigneten Fremdmaterial versehen ist. Solche Marker-Elemente können fest an der Punktionsnadel fixiert sein; alternativ können sie mit geeigneten Befestigungsmitteln in variablen Positionen längs der Punktionsnadel angebracht, beispielsweise angeklemmt werden. Insbesondere können diese Positionen im Zuge der erfindungsgemässen Verfahren berechnet werden. Vorteilhafterweise wird die Position eines dieser Marker-Elemente so gesetzt, dass es das obere oder gegebenenfalls das untere Plattenelement gerade dann erreicht, wenn die Spitze der Punktionsnadel zur Eingriffsregion gelangt. Sie wirken damit als Arretiermittel für den Vortrieb der Punktionsnadel (Anspruch 12).

Ein genereller Vorteil der vorliegenden Erfindung besteht darin, dass das Einführen und Positionieren einer Punktionsnadel unter Zuhilfenahme moderner Bildgebungsverfahren auf präzise und schnelle Art und Weise möglich ist. Daraus ergeben sich offensichtliche Vorteile für die Erfolgsrate und Sicherheit derartiger Eingriffe. Darüber hinaus wird die Eingriffszeit optimiert, was einerseits eine Kostenreduktion ergibt und zudem beispielsweise bei röntgenbasierten Methoden eine Reduktion der Strahlenbelastung ermöglicht.

### Kurzbeschreibung der Zeichnungen

Ausführungsbeispiele der Erfindung werden nachfolgend anhand der Zeichnungen näher beschrieben, dabei zeigen:
- Fig. 1: eine Einführungs- und Positioniervorrichtung, in perspektivischer Darstellung;
- Fig. 2: ein Durchgangsloch mit einer ersten Art von Anpassungsmittel, (a) ohne eingesetzte Punktionsnadel, und (b) mit eingesetzter Punktionsnadel, in einer Schnittdarstellung;
- Fig. 3: ein Durchgangsloch mit einer zweiten Art von Anpassungsmittel, (a) ohne eingesetzte Punktionsnadel, und (b) mit eingesetzter Punktionsnadel, in einer Schnittdarstellung;
- Fig. 4: ein Durchgangsloch mit einer dritten Art von Anpassungsmittel und eine anliegend durchgeführte Punktionsnadel, in einer Schnittdarstellung;
- Fig. 5: eine weitere Ausgestaltung der Einführungs- und Positioniervorrichtung, (a) in zusammengestecktem Zustand, und (b) in gelöstem und teilweise ausgeklapptem Zustand;
- Fig. 6: noch eine weitere Ausgestaltung der Einführungs- und Positioniervorrichtung, in gestapeltem Zustand und mit teilweise eingeführter Punktionsnadel, in perspektivischer Darstellung;
- Fig. 7: ein Plattenelement mit einer Ausgestaltung der Markierungsmittel, in Draufsicht;
- Fig. 8: ein weiteres Plattenelement mit einer anderen Ausgestaltung der Markierungsmittel, in Draufsicht; und
- Fig. 9: eine CT-Schnittbild einer Einführungs- und Positioniervorrichtung einschliesslich Punktionsnadel im Rahmen eines simulierten Eingriffs in der Lendenwirbelsäulenregion eines Phantoms.

Grundsätzlich werden identische oder gleichwirkende Komponenten in den verschiedenen Figuren mit denselben Bezugszeichen bezeichnet.

### Wege zur Ausführung der Erfindung

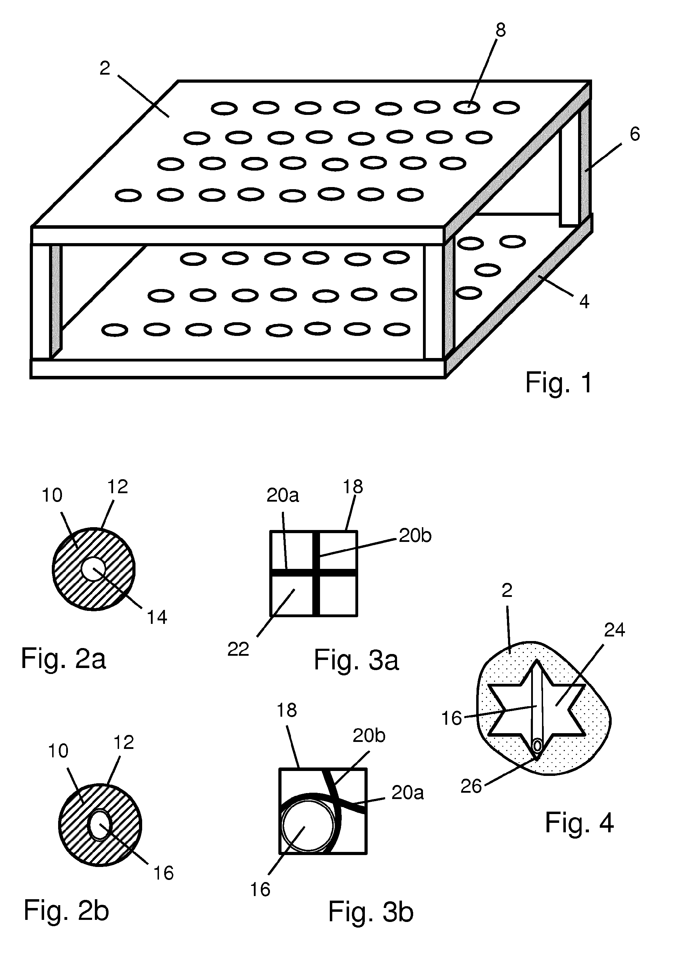

Die in der Fig. 1 dargestellte Einführungs- und Positioniervorrichtung weist ein oberes Plattenelement 2 und ein unteres Plattenelement 4 auf, welche mittels Verbindungselementen 6 starr voneinander beabstandet gehalten sind. Jedes Plattenelement ist mit einer Vielzahl von Durchgangslöchern 8 zur Durchführung einer hier nicht dargestellten Punktionsnadel vorgegebener Aussenabmessungen versehen.

Jedes Durchgangsloch ist mit Anpassungsmitteln versehen, um eine Querabmessung des Durchgangsloches an einen durch den gewählten Neigungswinkel definierten Aussenquerschnitt der Punktionsnadel anzupassen.

Eine erste Ausführungsform eines solchen Anpassungsmittels ist in den Fig. 2a und 2b dargestellt. Das Anpassungsmittel besteht aus einer ringförmigen Einlage 10 aus einem gummielastischen Material, die in einer kreisrunden Öffnung 12 im Plattenelement eingesetzt ist. Zwecks Fixierung an den Öffnungsrändern kann die Einlage mit oberen und unteren Lippenrändern versehen sein. Der mittige Bereich 14 der Einlage bildet das Durchgangsloch für eine Punktionsnadel. Wie aus der Fig. 2b ersichtlich, bewirkt eine eingesetzte Punktionsnadel 16 mit kreisförmigem Aussenprofil im Fall einer erheblichen Neigung eine elliptische Verformung des Durchgangsloches 14.

Die Fig. 3a und 3b zeigen eine weitere Ausführungsform eines Anpassungsmittels. Dieses wird im gezeigten Beispiel durch zwei kreuzförmig über ein quadratisches Durchgangsloch 18 gespannte elastische Bänder 20a und 20b gebildet, welche im Durchgangsloch vier Öffnungen 22 frei lassen. Wie aus der Fig. 3b ersichtlich, wird eine Punktionsnadel 16, die in eine der Öffnungen 22 eingeführt wird, durch eine laterale Spannkraft der Bänder 20a und 20b gehalten. Die bei Neigung der Punktionsnadel auftretende Querschnittsveränderung in der Plattenebene wird durch die Elastizität der Bänder kompensiert.

Die Fig. 4 zeigt noch eine weitere Ausführungsform eines Anpassungsmittels. Dieses wird durch ein sternförmiges Durchgangsloch 24 gebildet, welches im gezeigten Beispiel sechs Arme aufweist. Eine durch die Öffnung geführte Punktionsnadel 16 liegt in einer gewählten Eckregion 26 an.

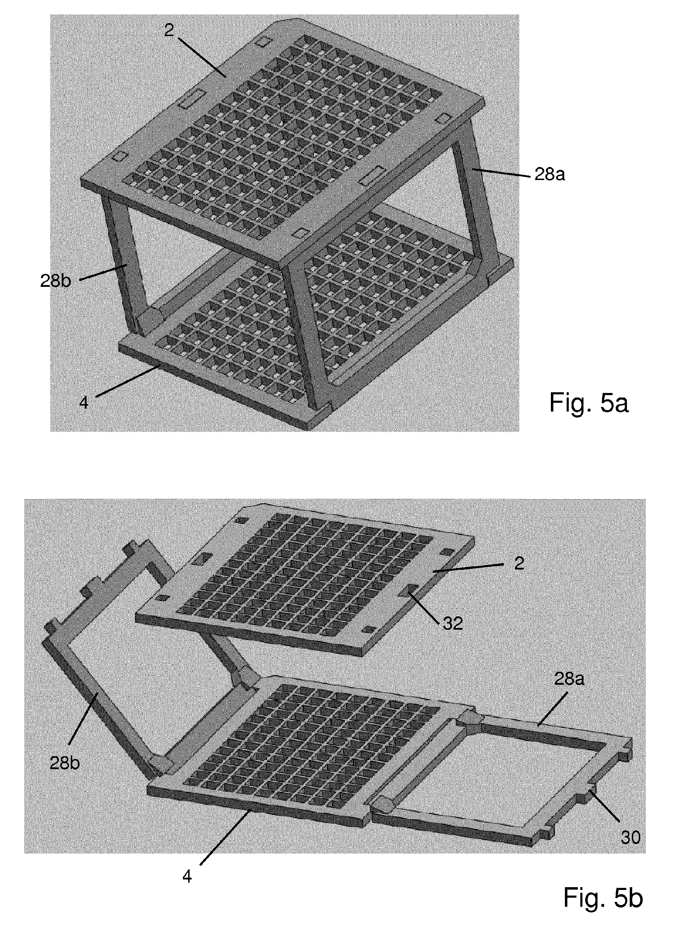

Bei der in den Fig. 5a und 5b gezeigten Ausgestaltung sind die Verbindungselemente zwischen dem oberen Plattenelement 2 und dem unteren Plattenelement 4 als rahmenförmige Teile 28a und 28b ausgestaltet. Jedes rahmenförmige Teil ist am unteren Plattenelement 4 nach aussen schwenkbar angelenkt und am oberen Plattenelement 2 mittels Vorsprüngen 30 in entsprechende Ausnehmungen 32 einsteckbar.

Bei einer ähnlichen, hier nicht dargestellten Ausgestaltung sind die Verbindungselemente am oberen Plattenelement mit einer seitlich einrastbaren Steckverbindung lösbar verbunden.

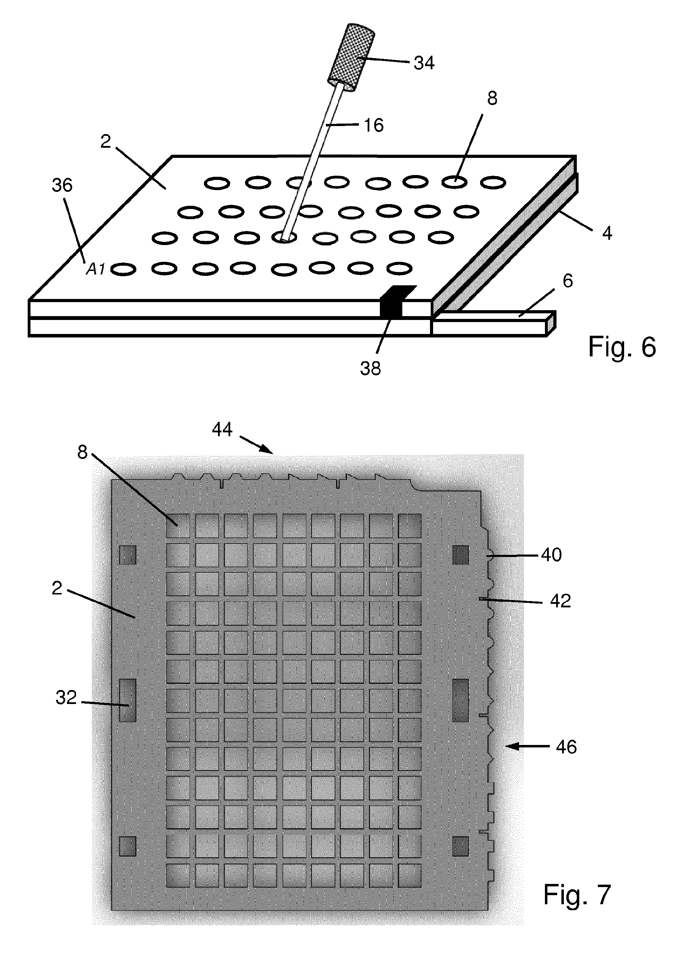

Weitere vorteilhafte Merkmale sind beispielhaft in der Fig. 6 dargestellt. Nach Entfernen bzw. Entkoppeln der Verbindungselemente 6 sind die beiden Plattenelemente 2 und 4 aufeinander gestapelt. Die bereits teilweise eingeführte Punktionsnadel 16 könnte nun weiter in den Körper vorgetrieben werden, bis ein Marker-Element 34 am oberen Plattenelement 2 anschlägt. Die einzelnen Durchgangslöcher sind mit visuellen Identifikationsmitteln 36 versehen, von denen hier stellvertretend eines (A1) dargestellt ist. Weiterhin ist stellvertretend eines von mehreren Markierungsmitteln 38 zur Bestimmung der räumlichen Lage und Ausrichtung der Einführungs- und Positioniervorrichtung dargestellt. Im gezeigten Beispiel ist dieses als kleiner metallischer Einsatz ausgebildet, der in das obere Plattenelement eingelegt ist.

Bei der in Fig. 7 dargestellten Ausgestaltung sind die Markierungsmittel als eine Anreihung von Vorsprüngen 40 und Ausnehmungen 42 in einer jeweiligen Seitenfläche des Plattenelementes 2 ausgestaltet. Dadurch, dass die von den Vorsprüngen und Ausnehmungen gebildeten Muster für die betreffende Seitenfläche 44 bzw. 46 charakteristisch ist, wird eine Identifikation des betreffenden Vorrichtungsteils im aufgenommenen Bild ermöglicht.

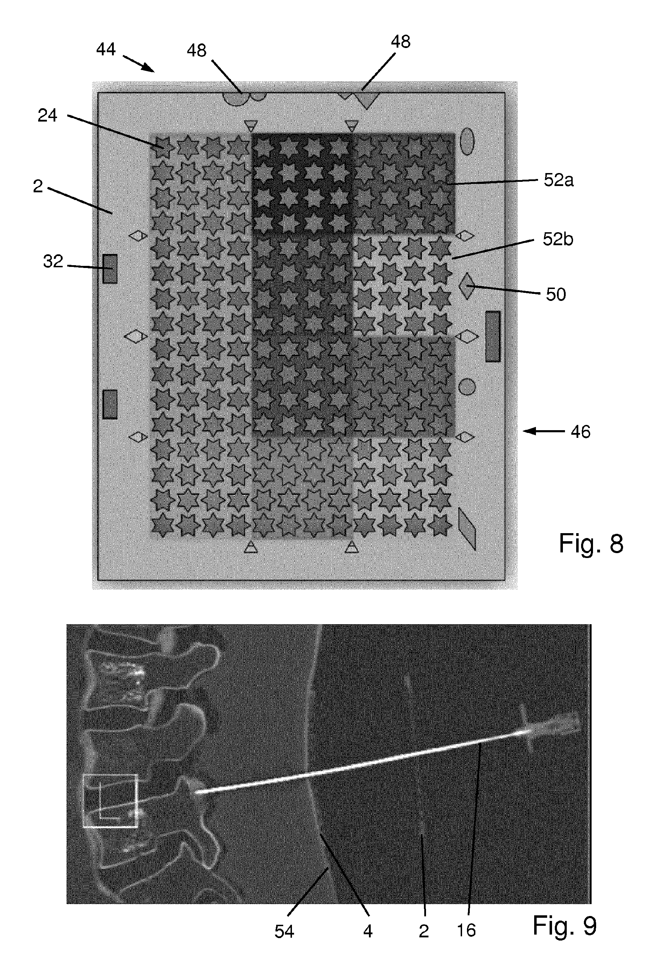

Die Fig. 8 zeigt eine weitere Ausgestaltung eines Plattenelementes 2, welches eine Anordnung von 12 x 16 sternförmigen Durchgangslöchern 24 aufweist. An der einen Seitenfläche 44 sind die Markierungsmittel als je zwei halbkreis- und dreieckförmige Ausnehmungen 48 realisiert. Zusätzlich sind noch zahlreiche weitere Markierungsmittel 50 mit rhomboidalem, dreieckförmigem, kreisrundem und elliptischem Querschnitt vorhanden. Wie aus der Fig. 8 ersichtlich, sind auf dem Plattenelement jeweils 4 mal 4 Durchgangslöcher grafisch durch eine visuelle Hervorhebung, hier eine Farbkodierung 52a, 52b, etc., zusammengruppiert. Die Farbkodierung kann als Bestandteil oder als Ergänzung der visuellen Identifikationsmittel für das schnelle und sichere Auffinden der von der Steuerungssoftware ausgegebenen Durchgangslöcher für eine Punktionsnadel verwendet werden.

### Ausführungsbeispiele

### Phantom-Anordnung

Eine Phantom-Anordnung, bestehend aus einer Metallkugel in einem Schaumstoffblock wurde in den Aufnahmebereich eines Siemens CT positioniert. Danach wurde eine Einführungs- und Positioniervorrichtung mit den nachfolgend angegebenen Spezifikationen auf die Oberseite des Schaumstoffblocks aufgebracht und mit den Klebelaschen fixiert.
- Material: Polyamid
- Abmessungen der Plattenelemente: 65 x 80 x 0.7 mm, versehen mit einem äquidistanten orthonormalen Muster von 12 x 16 Durchgangsöffnungen, Abstand zwischen den Plattenelementen: 45 mm.
- Als Anpassungsmittel dient eine 6-sternförmige Ausgestaltung der Durchgangslöcher.
- Als Punktionsnadel wurde eine handelsübliche 22g Nadel verwendet.

### Infiltrationstherapie an der Lendenwirbelsäule

In einem ersten Schritt wird ein Übersichtsbild im CT oder MRT akquiriert um sicherzustellen dass die Einführungs- und Positioniervorrichtung ("Punktionsbox") zum Zielpunkt im Körper dergestalt lokalisiert ist, dass der Zielpunkt erreicht werden kann.

Anschliessend wird unter sterilen Kautelen die Punktionsbox positioniert und befestigt. Es folgt nun der eigentliche 3D Planungsscan mit der Punktionsbox. Auf den Aufnahmen wählt nun der Arzt den exakten Zielpunkt im Körper (z.B. eine Gelenk zwischen Wirbelkörpern) und verbindet mit Hilfe der Software den Zielpunkt mit dem miterfassten unteren Plattenelement durch eine Gerade, diese definiert damit den unteren Plattendurchtritt, die Verlängerung dieser Geraden ergibt die Lokalisation des oberen miterfassten Plattenelementes. Die Punktionsnadel wird nun aufgrund dieser Angaben durch die daraus ermittelten Durchgangslöcher der Punktionsbox zum Ziel im Körper vorgeführt.

Die Fig. 9 zeigt ein entsprechendes Schnittbild an einem Phantom, in welchem eine Punktionsnadel 16, ein oberes Plattenelement 2, ein an der Körperoberfläche 54 angelegtes unteres Plattenelement 4 ersichtlich ist..

## Patentansprüche

1. Einführungs- und Positioniervorrichtung für mindestens eine Punktionsnadel, **gekennzeichnet durch** ein oberes Plattenelement (2) und ein unteres Plattenelement (4), welche mittels Verbindungselementen (6; 28a, 28b) starr voneinander beabstandet gehalten sind, wobei jedes Plattenelement mit einer Vielzahl von Durchgangslöchern (8; 18; 24) zur Durchführung einer Punktionsnadel (16) vorgegebener Aussenabmessungen versehen ist, wobei **durch** Wahl eines Durchgangsloches im unteren Plattenelement eine Einstichstelle und **durch** Wahl je eines Durchgangsloches im oberen und im unteren Plattenelement ein Neigungswinkel der Punktionsnadel wählbar sind, wobei ein jedes Durchgangsloch mit Anpassungsmitteln (10; 20a, 20b; 26) versehen ist, um eine Querabmessung des Durchgangsloches an einen **durch** den gewählten Neigungswinkel definierten Aussenquerschnitt der Punktionsnadel anzupassen, und wobei in einem tomografischen Bildgebungsverfahren erkennbare Markierungsmittel (38; 40, 42; 48, 50) zur Bestimmung der räumlichen Lage und Ausrichtung der Einführungs- und Positioniervorrichtung vorhanden sind.

2. Einführungs- und Positioniervorrichtung nach Anspruch 1, wobei ein jedes Anpassungsmittel als elastisches Element (10) im betreffenden Durchgangsloch ausgebildet ist.

3. Einführungs- und Positioniervorrichtung nach Anspruch 1 oder 2, wobei ein jedes Anpassungsmittel durch eine sternartige Ausgestaltung (24) des betreffenden Durchgangsloches gebildet wird.

4. Einführungs- und Positioniervorrichtung nach einem der Ansprüche 1 bis 3, wobei die Durchgangslöcher mit visuellen Identifikationsmitteln (36; 52a, 52b) versehen sind.

5. Einführungs- und Positioniervorrichtung nach einem der Ansprüche 1 bis 4, wobei zumindest das obere Plattenelement transparent ist.

6. Einführungs- und Positioniervorrichtung nach einem der Ansprüche 1 bis 5, wobei die Durchgangslöcher auf dem zugehörigen Plattenelement als äquidistantes, vorzugsweise orthonormales zweidimensionales Gitter angeordnet sind.

7. Einführungs- und Positioniervorrichtung nach einem der Ansprüche 1 bis 6, wobei die Verbindungselemente als Seitenwandelemente (28a, 28b) ausgebildet sind, welche optional mit Ausnehmungen versehen sind.

8. Einführungs- und Positioniervorrichtung nach einem der Ansprüche 1 bis 7, wobei die Verbindungselemente jeweils zumindest von einem der Plattenelemente lösbar sind derart, dass die Plattenelemente aufeinander stapelbar sind.

9. Einführungs- und Positioniervorrichtung nach einem der Ansprüche 1 bis 8, dass die Markierungsmittel als Vorsprünge und/oder Ausnehmungen (40, 42, 48) in einer jeweiligen Seitenfläche (44, 46) eines Plattenelementes und/oder als zusätzliche Durchgangslöcher (50) im Plattenelement ausgestaltet sind.

10. Einführungs- und Positioniervorrichtung nach einem der Ansprüche 1 bis 9, wobei Fixierungsmittel vorhanden sind, um die Vorrichtung an einem Patienten zu fixieren.

11. Bausatz zur Einführung und Positionierung mindestens einer Punktionsnadel, umfassend eine Einführungs- und Positioniervorrichtung nach einem der Ansprüche 1 bis 10 sowie mindestens eine Punktionsnadel, wobei die Punktionsnadel zumindest abschnittsweise aus einem im tomografischen Bildgebungsverfahren erkennbaren Material gebildet ist.

12. Bausatz nach Anspruch 11, mit einem an der Punktionsnadel fixierbaren Arretiermittel, welches als Anschlag gegen ein Plattenelement, vorzugsweise gegen das obere Plattenelement vorgesehen ist.

13. Verfahren zur Planung der Einführung und Positionierung mindestens einer Punktionsnadel in eine vordefinierte Eingriffsregion eines Patienten, die sich in einem Aufnahmebereich eines bildgebenden Tomografiegeräts befindet, wobei das Verfahren die folgenden Schritte umfasst:
a) Anordnen einer Einführungs- und Positioniervorrichtung nach einem der Ansprüche 1 bis 10 derart, dass sich die Vorrichtung in einer fixen Position und Ausrichtung gegenüber der Eingriffsregion befindet;
b) Erfassen einer dreidimensionalen Bilddarstellung mittels des Tomografiegeräts, welche zumindest die Eingriffsregion und die Markierungsmittel der Einführungs- und Positioniervorrichtung enthält;
c) rechnergestützte Bestimmung der relativen räumlichen Lage der Eingriffsregion sowie sämtlicher Durchgangslöcher;
d) rechnergestützte Bestimmung und Ausgabe von zielführenden Kombinationen jeweils eines ersten Durchgangsloches im oberen Plattenelement und eines zweiten Durchgangsloches im unteren Plattenelement, für welche eine die beiden Durchgangslöcher mittig durchlaufende Gerade auf die Eingriffsregion trifft; und
e) optional, rechnergestützte Bestimmung des jeweiligen Abstandes zwischen zweitem Durchgangsloch und Eingriffsregion.

14. Verfahren zur Einführung und Positionierung mindestens einer Punktionsnadel in eine vordefinierte Eingriffsregion eines Patienten, die sich in einem Aufnahmebereich eines bildgebenden Tomografiegeräts befindet, wobei das Verfahren die folgenden Schritte umfasst:
a) Durchführung des Planungsverfahrens nach Anspruch 13;
b) Auswahl einer zielführenden Kombination eines ersten Durchgangsloches im oberen Plattenelement und eines zweiten Durchgangsloches im unteren Plattenelement;
c) Einführung einer Punktionsnadel durch das erste und zweite Durchgangsloch, Einstich in eine an das zweite Durchgangsloch angrenzende Hautregion und Vortrieb der Punktionsnadel bis deren Spitze zur Eingriffsregion gelangt.

15. Verfahren nach Anspruch 14, wobei die Punktionsnadel zumindest abschnittsweise aus einem im tomografischen Bildgebungsverfahren erkennbaren Material gebildet ist und wobei der Schritt c) mittels eines tomografischen Bildgebungsverfahrens gesteuert wird.
